# EUROPEAN PATENT SPECIFICATION

(11) **EP 1 561 460 B1**
(45) Date of publication and mention of the grant of the patent: **20.12.2006**
(21) Application number: 03765124.7
(22) Date of filing: 16.07.2003
(51) Int. Cl.: A61K 9/51

(54) **NANOPARTICLES FOR THE ADMINISTRATION OF ACTIVE INGREDIENTS, METHOD OF PRODUCING SAID PARTICLES AND COMPOSITION CONTAINING SAME**
NANOTEILCHEN ZUR VERABREICHUNG VON WIRKSTOFFEN, VERFAHREN ZUR HERSTELLUNG DIESER TEILCHEN UND DIESE ENTHALTENDE ZUSAMMENSETZUNG
NANOPARTICULES POUR L'ADMINISTRATION DE PRINCIPES ACTIFS, PROCEDE D'ELABORATION DES DITES PARTICULES ET COMPOSITION LES CONTENANT

(30) Priority: 19.07.2002 ES 200201694
(43) Date of publication of application: 10.08.2005
(73) Proprietor: ADVANCELL - Advanced In Vitro Cell Technologies, 08028 Barcelona (ES)
(72) Inventor: ALONSO FERNANDEZ, Maria José Facultade de Farmacia, 15782 Santiago de Compostela (ES); REMUNAN LOPEZ, Carmen Facultade de Farmacia, 15782 Santiago de Compostela (ES); CUNA VILAN, Margarita Maria Facultade de Farmacia, 15782 Santiago de Compostela (ES); ALONSO SANDE, Maria Facultade de Farmacia, 15782 Santiago de Compostela (ES); VILA JATO, José Luis Facultade de Farmacia, 15782 Santiago de Compostela (ES)
(74) Representative: ABG Patentes, S.L.
(86) International application number: PCT/ES2003/000372
(87) International publication number: WO 2004/009060

(56) References cited:
- EP-A1- 0 860 166
- US-A- 5 562 924

## Description

### FIELD OF THE INVENTION

The present invention refers to nanoparticles comprising chitosan, glucomannan, at least one active ingredient and, if necessary, an anionic salt, preferably in sodium tripolyphosphate form, which can be used to administer active ingredients to the human or animal body. It further refers to a method for obtaining said nanoparticles, and to a composition comprising said nanoparticles.

### BACKGROUND OF THE INVENTION

It is known that the administration of a number of active ingredients by different administration routes to the human or animal body has various difficulties. It is especially worth indicating the difficulties of administration by mucosal routes, especially peptides and proteins, given that said administration is strongly affected by the limited permeability of the epithelial barriers of the human or animal body. It is also known that it is possible to overcome part of these difficulties by incorporating the active ingredients which are to be administered in small particles. The transport of said particles through the mucosae is affected mainly by the size of these particles, the transport increasing with the decrease of the particle size. Therefore, the transport of, for example, nanoparticles (generally, with a mean diameter of less than 1 µm), through mucosae is better than that of microparticles (generally, with a mean diameter of 1 µm up to several hundred µm). In fact, the transport of nanoparticles through the mucosae of the human or animal body occurs naturally. It is also known that the effectiveness of the interaction between the nanoparticles with the epithelial cells can be improved by means of the incorporation of the nanoparticles of materials containing specific ligands. For example, the absorption of liposomes by M cells can be improved by coating said liposomes with mannan residues, as disclosed in the documents *Takada et al., Biochim. Biophys. Acta 802, 1984, 237-243* and *Tomizawa et al., Pharm. Res. 10,* 1993, *549-552.* It is further known that nanoparticles constituted of chitosan foment the transport of macromolecules incorporated therein through the nasal and intestinal epithelium.

A series of publications are known in the State of the Art referring to chitosan nanoparticles for the administration of active ingredients and to methods for obtaining said nanoparticles. Among said publications, it is worth pointing out *Calvo et al., J. Appl. Polym. Sci., 1997, 63, 125-132; Calvo et al., 14, 1997b, 1431-1436; Femández-Urrusuno et al., Phar. Res. 16, 1991a, 1576-1581; Fernández -Urrusuno et al., S.T.P. Pharm. Sci. 9, 1999b, 429-436; Tokumitsu et al., Pharm. Res. 16, 1999, 1830-1835; Mitra et al., J. Control. Release 74*, *2001, 317-323; US-A-2001051189 and US-A-5,843,509.* All these nanoparticles, the main component of which is chitosan, have the drawback of not being stable at given pH values, specifically, they dissolve in an acid medium and precipitate in a basic medium.

Patent document *WO-A-01*/*01964* for its part refers to compositions for the sustained release of biomolecules, in microparticle form, comprising an anionic polymer and another cationic polymer, which interact with one another, and biomolecules. The cationic polymer can be a water soluble, positively charged polymer, such as chitosan, for example. Dextran sulfate, heparin, alginic acid, alginate, carrageenan, an anionic polymethacrylate and a positively charged polyamino acid are mentioned as anionic polymers.

Patent document *WO-A-96*/*20698* refers to nanoparticles for the sustained release of bioactive agents, comprising a core based on a biocompatible and biodegradable polymer, which can be chitosan, said nanoparticles having associated or incorporated at least one bioactive agent and at least one surface modifying agent. It also refers to a method for obtaining the nanoparticles, based on the mixture of organic solutions of the components, and their subsequent addition to an aqueous phase, with subsequent evaporation of the organic solvent and separation of the nanoparticles from the resulting aqueous phase.

Patent document *WO-A-01*/*32751* refers to a method for producing chitosans or chitosan derivatives in nanoparticle form, with a mean particle diameter in the range of 10 to 1000 nm, consisting of dissolving the chitosan or chitosan derivative in an aqueous acid medium and raising the pH of the solution in the presence of a surface modifying agent, until the precipitation of the chitosan is achieved.

Patent document *WO-A-99*/*47130* refers to nanoparticles having a biocompatible and biodegradable polyelectrolyte complex, starting from at least one polycation (which can be chitosan) and at least one polyanion, as well as at least one bioactive ingredient, the nanoparticles being obtainable by additionally treating the polyelectrolyte complex during or after its formation with at least one cross-linking agent (glyoxal, TSTU or EDAP).

EP-A-0 860 166 discloses nanoparticles comprising chitosan and polyethylen oxide.

Most known nanoparticle and microparticle systems produced on the basis of chitosan have the important drawback of being unstable after their administration in *vivo,* as well as during the storage thereof. There are usually difficulties in the lyophilization process, specifically difficulties in the reconstitution of the lyophilized systems, which represents an important additional limitation for the suitable exploitation of this type of systems. In addition, for the lyophilization of these systems, disclosed in the state of the art, it is necessary to add high amounts of sugars. As a result of the foregoing, the known nanoparticles and microparticles must generally be stored in a liquid suspension form; this usually results in the destruction of these systems in a few months.

For its part, glucomannan has traditionally been used as a diet supplement, for the purpose of reducing cholesterol level, in addition to the fact that it has been used in cosmetic applications. The use of glucomannan in the preparation of pharmaceutical compositions is disclosed in a series of documents. For example, its use in the preparation of pharmaceutical compositions in gel form is mentioned in *WO-A-99*/*01166*, and *US-A-5,662,840; Xiao et al., J. Appl. Polym. Sci. 76, 2000, 509-515* and *US-A-6,159,504* mention their use in pharmaceutical compositions in film form; *US-A-2002019447* and *US-A-2002018812* mention pharmaceutical compositions in foam, capsule and sponge form; and *US-A-6,221,393* in compositions in tablet form. Some of these documents mention the possible incorporation of chitosan to the pharmaceutical compositions.

It has been mentioned in some documents the possible existence of an interaction between chitosan and glucomannan, for example in film form, in *Xiao et al., J. Appl. Polym. Sci. 76, 2000, 509-515,* as well as in granule form with a diameter exceeding 1 mm, for administering analgesic drugs, in *Xie et al., J. Macromol. Sci., Pure Appl. Chem. A29, 1992, 931-8* and *Xie et al., J. Clin. Pharm. Sci. 1, 1992, 42-8*. German publication DE19839515 refers to a pharmaceutical preparation containing at least one polymer-colloidal active ingredient (particle size < 1 µm) association product, in which at least one component is a biocompatible and biodegradable polymer, which can be chitosan.

In accordance with all the foregoing, there is a need to provide a type of nanoparticles with improved properties of absorption by the human or animal body, especially through the mucosal tissue, and which could be stored for a longer time period without undergoing important alterations. Furthermore, the methods for preparing nanoparticles, whether based on chitosan or not, usually require the use of organic solvents, which have the drawbacks known by any person skilled in the art, such as their toxicity and the difficulty of eliminating them from the nanoparticles, whereby it is appropriate to find a method for producing nanoparticles complying with the above-mentioned properties, which is simple and does not require the use of organic solvents.

### DESCRIPTION OF THE INVENTION

It has now been found that nanoparticles comprising chitosan, glucomannan, the active ingredient to be administered and, optionally, an anionic salt, preferably sodium tripolyphosphate, solve the mentioned drawbacks of the state of the art. A simple method of preparing the previous nanoparticles, without the need of using organic solvents, has further been found.

According to a first aspect, the present invention refers to a method for producing nanoparticles with a mean diameter of less than or equal to 1 µm, incorporating at least one active ingredient, comprising the following steps:
a) preparing an aqueous chitosan solution,
b) preparing an aqueous glucomannan solution, and
c) mixing, under stirring, the solutions of steps a) and b), such that the chitosan and glucomannan nanoparticles are obtained,
wherein at least one of the solutions of steps a) and b) contains at least one active ingredient.

According to a second aspect, the present invention refers to nanoparticles obtained according to the previous method, comprising chitosan, glucomannan and at least one active ingredient.

According to an additional aspect, the invention refers to a pharmaceutical or cosmetic composition comprising the previous nanoparticles, together with at least one pharmaceutically or cosmetically acceptable excipient, respectively.

According to a preferred embodiment of the method, the glucomannan solution further contains an anionic salt, preferably in tripolyphosphate sodium form, for the purpose of favoring the spontaneous formation of the nanoparticles.

Preferably, the concentration of the chitosan solution used in the method is in the range between 0.5 and 5 mg/mL.

Also preferably, the concentration of the glucomannan solution of the method is in the range between 0.1 and 50 mg/mL.

The ratio of chitosan with regard to glucomannan (by weight) may vary, preferably between 1:0.02 to 1:100, particularly preferred between 1:0.5 and 1:50.

During the method of producing the nanoparticles, the pH value of the chitosan solution is preferably maintained at a pH between 2 and 6.

The method of producing the chitosan and glucomannan nanoparticles can further comprise an additional step, in which said nanoparticles are lyophilized. Unlike in the nanoparticles known in the state of the art, for the lyophilization of the nanoparticles according to the present invention, only the addition of small amounts of sugars is needed, although it is also possible to carry out the lyophilization without adding sugars. In their lyophilized form, the nanoparticles can be stored for longer periods of time, and they can be easily regenerated, when needed, by means of adding the necessary amount of water.

According to this additional embodiment in which the obtained nanoparticles are lyophilized, the present invention further refers to the chitosan and glucomannan nanoparticles, according to the invention, lyophilized, and to a pharmaceutical or cosmetic composition comprising said lyophilized nanoparticles, and at least one pharmaceutically or cosmetically acceptable excipient.

The chitosan and glucomannan nanoparticles obtainable by means of the method described above have better stability upon contact with biological fluids and also during storage than the nanoparticles known in the state of the art. In fact, the chitosan and glucomannan nanoparticles are stable in both an aqueous acid and basic medium, whereby they can be stored in liquid suspension form for long periods of time. They also have an improved capacity of absorption by the human or animal body.

Additionally, the chitosan and glucomannan nanoparticles are systems of both pharmaceutical and cosmetic utility. They can further be administered by several routes, such as, for example, the topical, oral, nasal, pulmonary, vaginal and subcutaneous routes. The active ingredient to be incorporated in the chitosan and glucomannan nanoparticles is the ingredient for which the formulation is intended. This ingredient will have an effect on the human or animal organism after its administration; said effect may cure, minimize or prevent a disease. The active ingredient can be a drug, a vitamin, a vaccine, etc., or a cosmetic agent, intended for improving the physical and aesthetic appearance (for example, skin moisturizing).

The chitosan and glucomannan nanoparticles according to the present invention have a high capacity of association of bioactive macromolecules, for example insulin, bovine serum albumin or immunogenic proteins. The capacity of association depends on the type of macromolecule incorporated. Likewise, for certain macromolecules, the degree of association may depend on the deacetylation degree of the chitosan: the higher the deacetylation degree, the greater the effectiveness of association.

However, it is also possible to incorporate other active ingredients to the nanoparticles, both of a lipophilic and hydrophilic nature. It is possible to point out, for example, the effective incorporation of indomethacin (moderately lipophilic) and acyclovir (hydrophilic).

The active ingredient to be incorporated in the nanoparticles is dissolved in one of the two aqueous solutions used in the formation of the nanoparticles. In the case of lipophilic active ingredients, these must be previously dissolved in a polar organic solvent, miscible with aqueous media, and then it will be added to the chitosan solution, or to the glucomannan solution. In the case of incorporating more than one active ingredient to the nanoparticles, these can be dissolved either in the same solution or in different solutions.

In the case of incorporating bioactive macromolecules, these can preferably be incorporated according to the following methods:
i) the macromolecule is dissolved in sodium tripolyphosphate, and the resulting mixture is incorporated to the glucomannan solution used for producing the nanoparticles;
ii) the macromolecule is dissolved in a NaOH solution; the obtained solution is added to sodium tripolyphosphate; the resulting mixture is incorporated to the glucomannan solution used for producing the nanoparticles;
iii) the macromolecule is dissolved in sodium phosphate at pH 6.6; the obtained solution is added to sodium tripolyphosphate; the resulting mixture is incorporated to the glucomannan solution used for producing the nanoparticles;
iv) the macromolecule is added to the chitosan solution used for producing the nanoparticles;
v) the macromolecule is added to a NaOH solution; the obtained mixture is added to the chitosan solution used for producing the nanoparticles;
vi) the macromolecule is added to a sodium phosphate solution at pH 6.6; the obtained mixture is added to the chitosan solution used for producing the nanoparticles.

The chitosan and glucomannan nanoparticles are colloidal, i.e. their mean diameter is equal to or less than 1 µm. The mean particle size is mainly affected by the ratio of chitosan with regard to glucomannan, by the deacetylation degree of the chitosan, by the incorporation of an anionic salt, for example sodium tripolyphosphate, and by the nature of the active ingredient. On the other hand, the nanoparticles can have a positive or negative surface charge (measured by means of the zeta potential), the magnitude of which depends on the composition of the nanoparticles and the deacetylation degree of the chitosan.

The nanoparticles further have the capacity to release the active ingredient incorporated therein in a sustained and/or controlled manner. The release of the active ingredient can be controlled by combining factors such as the ratio of chitosan with regard to glucomannan, the degree of acetylation of the chitosan and the method of producing the nanoparticles.

Other purposes, features and advantages of the invention will become clearer below in light of the explanatory description which follows, made in reference to several illustrative examples.

### EXAMPLES:

Through out the examples, the following abbreviations will be sued:
CS = Chitosan
GM = Phosphorylated glucomannan
TPP = Sodium tripolyphosphate
P1: Plant origin protein (*Ricinus communis*) constituted of two polypeptides.

### Example 1

Chitosan (with an 88% deacetylation degree), glucomannan and sodium tripolyphosphate nanoparticles were prepared according to the method of the invention, with different glucomannan ratios. Once they were prepared, their mean diameter and zeta potential were measured.

**Table 1**

| CS/TPP/GM (w/w) | Mean diameter (nm) | Zeta potential (mV) |
|---|---|---|
| 6/1/2.3 | 250 ± 24 | + 32.2 ± 2.0 |
| 6/1/4.6 | 302 ± 26 | +15.2 ± 1.7 |

### Example 2

Chitosan (with an 88% deacetylation degree) and glucomannan nanoparticles were prepared according to the method of the invention, with different glucomannan ratios and without adding any anionic salt. Once they were prepared, their mean diameter and Z potential were measured.

**Table 2**

| CS/GM (w/w) | Mean diameter (nm) | Zeta potential (mV) |
|---|---|---|
| 6/4.6 | 252.1 ± 15 | + 31.25 ± 1.06 |
| 6/13.8 | 185.5 ± 3 | +33.2 ± 0.8 |

### Example 3

Chitosan (with an 88% deacetylation degree) and glucomannan nanoparticles were prepared, incorporating sodium tripolyphosphate, according to the method of the invention, with different chitosan and glucomannan ratios, incorporating the P1 protein or insulin. Once they were prepared, their mean diameter and zeta potential were measured.

**Table 3**

| CS/TPP/GM (w/w/w) | Associated protein | CS/protein (w/w) | Mean diameter (nm) | Zeta potential (mV) |
|---|---|---|---|---|
| 4/1/1.5 | P1 | 1.6/1 | 552 ± 4 | +32.1 ± 1 |
| 6/1/4.6 | P1 | 1.3/1 | 296 ± 6 | + 15.9 ± 0.2 |
| 6/1/4.6 | P1 | 2.2/1 | 263 ± 6 | +30.3 ± 0.6 |
| 6/0.7/4.6 | Insulin | 2/1 | 265.8 ± 6 | +32.2 ± 0.4 |

### Example 4

Chitosan (with an 88% deacetylation degree) and glucomannan nanoparticles were prepared, without incorporating any anionic salt, according to the method of the invention, with different chitosan and glucomannan ratios, incorporating the P1 protein or insulin. Once they were prepared, their mean diameter and zeta potential were measured.

**Table 4**

| CS/GM (w/w) | Associated protein | Mean diameter (nm) | Zeta potential (mV) |
|---|---|---|---|
| 6/4.6 | Insulin | 252.3 ± 4 | + 15.5 ± .06 |
| 6/4.6 | P1 | 205 ± 11 | + 8.5 ± 2.7 |
| 6/13.8 | P1 | 293.2 ± 5 | + 11.9 ± 3.2 |

### Example 5

Chitosan (88% deacetylation degree) and glucomannan nanoparticles were prepared, incorporating sodium tripolyphosphate, according to the method of the invention, with a CS/TPP ratio of 3:1, incorporating the P1 protein (25% theoretical load) therein, according to different methods. The effectiveness of association of the P1 protein to the nanoparticles, as well as the load capacity thereof, were measured.

**Table 5**

| Method | Effectiveness of association (%) | Load capacity (%) |
|---|---|---|
| TPPⁱ | 15.4 ± 2.7 | 8.1 ± 1.4 |
| NaOH-TPPⁱⁱ | 5.6 ± 0.3 | 3.2 ± 0.2 |
| Phosphate-TPPⁱⁱⁱ | 22.6 ± 0.2 | 11.1 ± 0.1 |
| CS^{iv} | 15.5 ± 2.7 | 7.1 ± 1.2 |
| NaOH-CS^{v} | 8.7 ± 3.2 | 5.1 ± 1.8 |
| Phosphate-CS^{vi} | 26.0 ± 0.1 | 11.9 ± 0.5 |

| | | |
|---|---|---|
| P1 was dissolved in ⁱTPP; ⁱⁱNaOH, and added to TPP ⁱⁱⁱsodium phosphate at pH 6.6, and added to TPP ^{iv}CS; ^{v}NaOH, and added to CS; and ^{vi}sodium phosphate at pH 6.6, and added to CS | | |

### Example 6

Chitosan (88% deacetylation degree) and glucomannan nanoparticles were prepared, incorporating sodium tripolyphosphate, according to the method of the invention, with a CS/TPP ratio of 6:1, incorporating P1 protein (25% theoretical load) therein, according to different methods. The effectiveness of association of the P1 protein to the nanoparticles, as well as the load capacity thereof, were measured.

**Table 6**

| Method | Effectiveness of association (%) | Load capacity (%) |
|---|---|---|
| TPPⁱ | 15.4 ± 4.0 | 16.8 ± 4.9 |
| NaOH-TPPⁱⁱ | 8.7 ± 1.6 | 10.5 ± 1.9 |
| Phosphate-TPPⁱⁱⁱ | 26.3 ± 1.2 | 27.6 ± 1.3 |
| CS^{iv} | 6.5 ± 1.7 | 9.8 ± 2.6 |
| NaOH-CS^{iv} | 18.2 ± 1.9 | 21.4 ± 2.4 |
| Phosphate-CS^{vi} | 24.5 ± 1.6 | 25.4 ± 1.7 |

| | | |
|---|---|---|
| P1 was dissolved in ⁱTPP; ⁱⁱNaOH, and added to TPP ⁱⁱⁱsodium phosphate at pH 6.6, and added to TPP ^{iv}CS; ^{v}NaOH, and added to CS; and ^{vi}sodium phosphate at pH 6.6, and added to CHITOSAN | | |

### Example 7

Chitosan (88% deacetylation degree) and glucomannan nanoparticles were prepared, without incorporating any anionic salt, according to the method of the invention, with different CS/GM ratios, incorporating P1 protein therein. The effectiveness of association of the P1 protein to the nanoparticles, as well as the load capacity thereof, were measured.

**Table 7**

| CS/GM (w/w) | Associated protein | Effectiveness of association (%) | Load capacity (%) |
|---|---|---|---|
| 6/4.6 | P1 | 37.3 ± 3.8 | 19.16 ± 1.95 |
| 6/13.8 | P1 | 22.4 ± 7.1 | 4.46 ± 1.41 |

### Example 8

Chitosan (88% deacetylation degree) and glucomannan nanoparticles were prepared, without incorporating any anionic salt, according to the method of the invention, incorporating P1 protein or insulin therein. The effectiveness of association of the P1 protein and insulin to the nanoparticles, as well as the load capacity thereof, were measured.

**Table 8**

| CS/GM (w/w) | Associated protein | Effectiveness of association (%) | Load capacity (%) |
|---|---|---|---|
| 6/4.6 | P1 | 37.3 ± 3.8 | 19.16 ± 1.95 |
| 6/4.6 | Insulin | 37.5 ± 3.1 | 23.77 ± 1.78 |

### Example 9

Chitosan (88% deacetylation degree) and glucomannan nanoparticles were prepared, according to the method of the invention, incorporating indomethacin or acyclovir therein. The effectiveness of association of indomethacin and of acyclovir to the nanoparticles, as well as the diameter thereof, were measured.

**Table 9**

| Associated drug | Effectiveness of association (%) | Mean diameter (nm) |
|---|---|---|
| Indomethacin | 81.81 ± 3.89 | 619 ± 15 |
| Acyclovir | 27.36 ± 7.90 | 304 ± 8 |

### Example 10

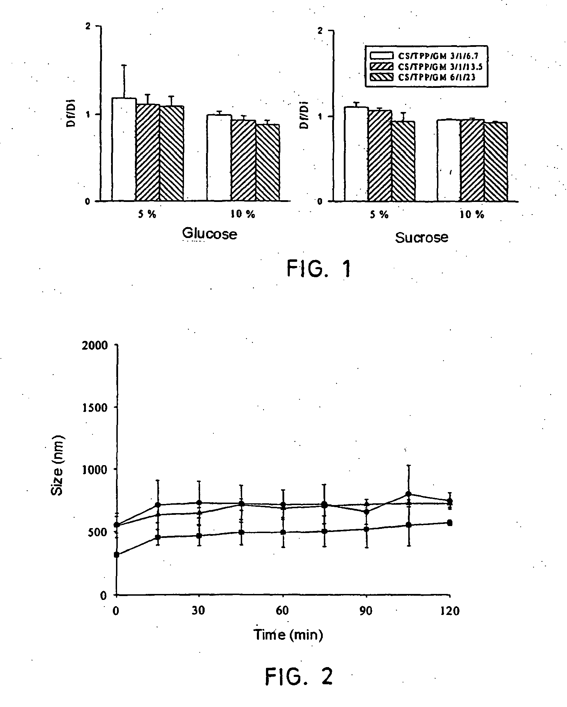

Chitosan (88% deacetylation degree), glucomannan and sodium tripolyphosphate nanoparticles were prepared, according to the method of the invention, with different CS/TPP/GM ratios. The effect thereon of the type and the concentration of the cryoprotective agent used in the lyophilization of the nanoparticles on the particle size and zeta potential have been checked (Df: final diameter, Di: initial diameter). (See Figure 1).

### Example 11

Chitosan (88% deacetylation degree), glucomannan and sodium tripolyphosphate nanoparticles were prepared, according to the method of the invention, with different CS/TPP/GM ratios. During their incubation in a phosphate buffer solution at pH 7.4 for 2 hours, the mean diameter of the particles was measured. Theoretical CS/TPP/GM ratios: (★) 3/1/13.5, (■) 3/1/6.7 and (-) 6/1/23: (See Figure 2)

### Example 12

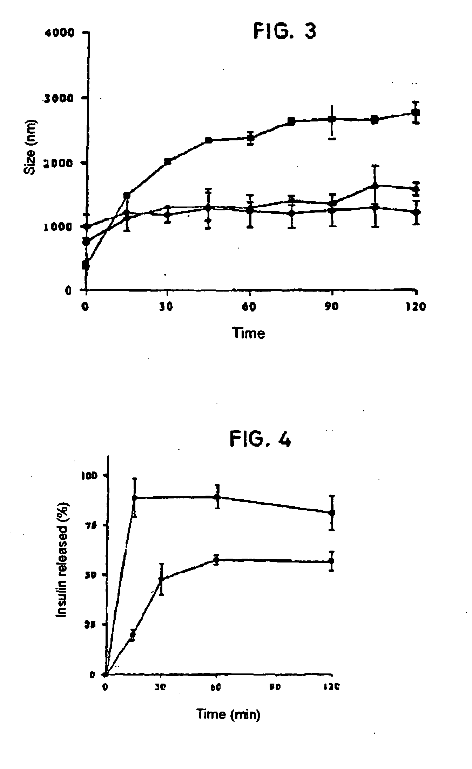

Chitosan (88% deacetylation degree) and glucomannan nanoparticles were prepared, according to the method of the invention, with different CS/GM ratios. During their incubation in a phosphate buffer solution at pH 7.4, the mean diameter of the particles was measured. (~) CS/GM = 6/4.6; (■) CS/GM = 6/13.8; (■) CS/TPP/GM = 6/1/4.6. (See Figure 3)

### Example 13

Chitosan (88% deacetylation degree) and glucomannan nanoparticles were prepared, according to the method of the invention, with different CS/GM ratios, incorporating insulin. The release of the insulin in phosphate buffer at pH 7.4 and 37°C was measured. (★) CS/TPP/GM = 6/1/4.6 and (■) CS/GM = 6/4.6. (See Figure 4)

### Example 14

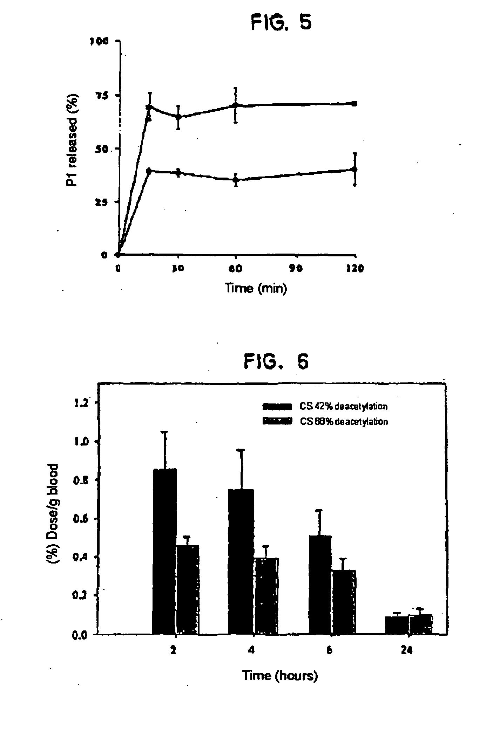

Chitosan (88% deacetylation degree) and glucomannan nanoparticles were prepared, according to the method of the invention, with different CS/GM ratios, incorporating P1 protein. The release of the insulin in phosphate buffer at pH 7.4 and 37°C was measured. (★) CS/TPP/GM = 6/1/4.6 and (■) CS/GM = 6/4.6. (See Figure 5)

### Example 15

Chitosan (42% and 88% deacetylation degree) and glucomannan nanoparticles were prepared, according to the method of the invention, incorporating ¹²⁵I-BSA. The nanoparticles were orally administered to mice, and blood radioactivity levels were measured after 2, 4, 6 and 24 hours. (See Figure 6)

### Example 16

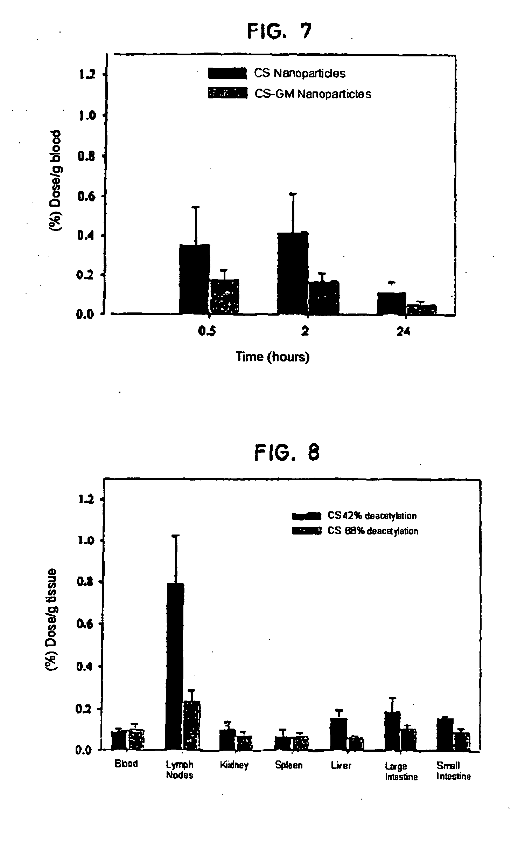

Chitosan and glucomannan nanoparticles were prepared, according to the method of the invention, incorporating ¹²⁵I-BSA. The nanoparticles were intraduodenally administered to mice, and blood radioactivity levels were measured after 0.5, 2 and 24 hours. Chitosan nanoparticles were used as a control. (See Figure 7)

### Example 17

Chitosan (42% and 88% deacetylation degree) and glucomannan nanoparticles were prepared, according to the method of the invention, incorporating ¹²⁵I-BSA. The nanoparticles were orally administered to mice, and radioactivity levels in different tissues were measured after 24 hours. (See Figure 8)

### Example 18

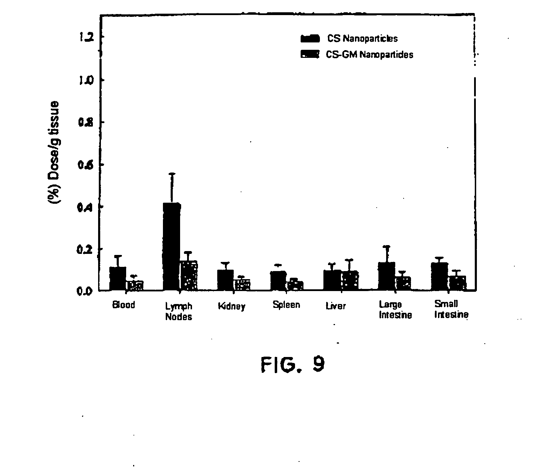

Chitosan and glucomannan nanoparticles were prepared, according to the method of the invention, incorporating ¹²⁵I-BSA. The nanoparticles were intraduodenally administered to mice, and radioactivity levels in different tissues were measured after 24 hours. Chitosan nanoparticles were used as a control. (See Figure 9)

## Claims

1. A method of producing nanoparticles, with a mean diameter equal to or less than 1 µm, and incorporating at least one active ingredient, **characterized in that** it comprises the following steps:
a) preparing an aqueous chitosan solution,
b) preparing an aqueous glucomannan solution, and
c) mixing, under stirring, the solutions of steps a) and b), such that the chitosan and glucomannan nanoparticles are obtained,
wherein at least one of the solutions of steps a) and b) contains at least one active ingredient.

2. A method of producing nanoparticles according to claim 1, **characterized in that** the glucomannan solution contains an anionic salt.

3. A method of producing nanoparticles according to claim 2, **characterized in that** the anionic salt is sodium tripolyphosphate.

4. A method of producing nanoparticles according to claim 3, **characterized in that** the sodium tripolyphosphate is at a concentration between 0.1 and 5 mg/mL.

5. A method of producing nanoparticles according to any of claims 1 to 4, **characterized in that** the concentration of the chitosan solution is in the range between 0.5 and 5 mg/mL.

6. A method of producing nanoparticles according to any of claims 1 to 5, **characterized in that** the concentration of the glucomannan solution is in the range between 0.5 and 50 mg/mL.

7. A method of producing nanoparticles according to any of claims 1 to 4, **characterized in that** the ratio between chitosan and glucomannan is between 1:0.1 and 1:100.

8. A method of producing nanoparticles according to any of claims 1 to 4 and 7, **characterized in that** the ratio between chitosan and glucomannan is between 1:0.5 and 1:50.

9. A method of producing nanoparticles according to any of claims 1 to 8, **characterized in that** the chitosan solution has a pH between 2 and 6.

10. A method of producing nanoparticles according to any of claims 1 to 9, **characterized in that** the active ingredient is a bioactive macromolecule.

11. A method of producing nanoparticles according to any of claims 1 to 10, **characterized in that** the active ingredient is chosen from the group comprising insulin, bovine serum albumin and immunogenic proteins.

12. A method of producing nanoparticles according to any of claims 1 to 9, **characterized in that** the active ingredient is a low molecular weight drug.

13. A method of producing nanoparticles according to any of claims 1 to 9 and 12, **characterized in that** the active ingredient is chosen from the group comprising acyclovir and indomethacin.

14. A method of producing nanoparticles according to any of claims 1 to 13, **characterized in that** it comprises an additional step after step c), in which the nanoparticles are lyophilized.

15. Nanoparticles with a diameter equal to or less than 1 µm, for the administration of at least one active ingredient, **characterized in that** they comprise chitosan, glucomannan and at least one active ingredient.

16. Nanoparticles according to claim 15, **characterized in that** they are obtainable by means of the method according to claims 1 to 11.

17. Nanoparticles according to any of claims 15 and 16, **characterized in that** they further comprise an anionic salt.

18. Nanoparticles according to claim 17, **characterized in that** the anionic salt is sodium tripolyphosphate.

19. Nanoparticles according to any of claims 15 to 18, **characterized in that** the active ingredient is a bioactive macromolecule.

20. Nanoparticles according to any of claims 15 to 19, **characterized in that** the active ingredient is selected from the group comprising insulin, bovine serum albumin and immunogenic proteins.

21. Nanoparticles according to any of claims 15 to 18, **characterized in that** the active ingredient is a drug of low molecular weight.

22. Nanoparticles according to any of claims 15 to 18 and 21, **characterized in that** the active ingredient is selected from the group comprising acyclovir and indomethacin.

23. Nanoparticles according to any of claims 15 to 22 **characterized in that** the chitosan:glucomannan ratio is between 1:0.02 and 1:100.

24. Nanoparticles according to any of claims 15 to 23, **characterized in that** the chitosan:glucomannan ratio is between 1:0.5 and 1:50.

25. Nanoparticles according to any of claims 15 to 22, **characterized in that** they are lyophilized after they are obtained.

26. A pharmaceutical composition, **characterized in that** it comprises the nanoparticles according to any of claims 15 to 24 and at least one pharmaceutically acceptable excipient.

27. A cosmetic composition, **characterized in that** it comprises the nanoparticles according to any of claims 15 to 24 and at least one cosmetically acceptable excipient.

28. A pharmaceutical composition, **characterized in that** it comprises the nanoparticles of claim 25, after being regenerated by means of the addition of water, and at least one pharmaceutically acceptable excipient.

29. A cosmetic composition, **characterized in that** it comprises the nanoparticles of claim 25, after being regenerated by means of the addition of water, and at least one cosmetically acceptable excipient.

## Patentansprüche

1. Verfahren zur Herstellung von Nanoteilchen mit einem mittleren Durchmesser, der 1 µm entspricht oder geringer ist und die mindestens einen Wirkstoff inkorporieren, **dadurch gekennzeichnet, dass** es die folgenden Schritte umfasst:
a) Herstellung einer wässrigen Chitosanlösung,
b) Herstellung einer wässrigen Glucomannanlösung und
c) Vermischen der Lösungen der Schritte a) und b) unter Rühren, so dass Chitosan- und Glucomannan-Nanoteilchen erhalten werden,
wobei mindestens eine der Lösungen gemäß den Schritten a) und b) mindestens einen wirkstoff enthält.

2. Verfahren zur Erzeugung von Nanoteilchen gemäß Anspruch 1, **dadurch gekennzeichnet, dass** die Glucomannanlösung ein anionisches Salz enthält.

3. Verfahren zur Erzeugung von Nanoteilchen gemäß Anspruch 2, **dadurch gekennzeichnet, dass** das anionische Salz Natriumtripolyphosphat ist.

4. Verfahren zur Erzeugung von Nanoteilchen gemäß Anspruch 3, **dadurch gekennzeichnet, dass** das Natriumtripolyphosphat in einer Konzentration von 0,1 bis 5 mg/ml vorliegt.

5. Verfahren zur Erzeugung von Nanoteilchen gemäß einem der Ansprüche 1 bis 4, **dadurch gekennzeichnet, dass** die Konzentration der Chitosanlösung in einem Bereich zwischen 0,5 und 5 mg/ml liegt.

6. Verfahren zur Erzeugung von Nanoteilchen gemäß einem der Ansprüche 1 bis 5, **dadurch gekennzeichnet, dass** die Konzentration der Glucomannanlösung in einem Bereich zwischen 0,5 und 50 mg/ml liegt.

7. Verfahren zur Erzeugung von Nanoteilchen gemäß einem der Ansprüche 1 bis 4, **dadurch gekennzeichnet, dass** das Verhältnis zwischen Chitosan und Glucomannan zwischen 1:0,1 und 1:100 liegt.

8. Verfahren zur Erzeugung von Nanoteilchen gemäß einem der Ansprüche 1 bis 4 und 7, **dadurch gekennzeichnet, dass** das Verhältnis zwischen Chitosan und Glucomannan zwischen 1:0,5 und 1:50 liegt.

9. Verfahren zur Erzeugung von Nanoteilchen gemäß einem der Ansprüche 1 bis 8, **dadurch gekennzeichnet, dass** die Chitosanlösung einen pH zwischen 2 und 6 aufweist.

10. Verfahren zur Erzeugung von Nanoteilchen gemäß einem der Ansprüche 1 bis 9, **dadurch gekennzeichnet, dass** der Wirkstoff ein bioaktives Makromolekül ist.

11. Verfahren zur Erzeugung von Nanoteilchen gemäß einem der Ansprüche 1 bis 10, **dadurch gekennzeichnet, dass** der Wirkstoff aus der Gruppe gewählt wird, umfassend Insulin, Rinderserumalbumin und immunogene Proteine.

12. Verfahren zur Erzeugung von Nanoteilchen gemäß einem der Ansprüche 1 bis 9, **dadurch gekennzeichnet, dass** der Wirkstoff ein niedermolekulares Arzneimittel ist.

13. Verfahren zur Erzeugung von Nanoteilchen gemäß einem der Ansprüche 1 bis 9 und 12, **dadurch gekennzeichnet, dass** der Wirkstoff gewählt ist aus der Gruppe, umfassend Acyclovir und Indomethacin.

14. Verfahren zur Erzeugung von Nanoteilchen gemäß einem der Ansprüche 1 bis 13, **dadurch gekennzeichnet, dass** es einen zusätzlichen Schritt nach Schritt c) umfasst, worin die Nanoteilchen lyophilisiert werden.

15. Nanoteilchen mit einem Durchmesser von 1 µm oder weniger für die Verabreichung von mindestens einem aktiven Wirkstoff, **dadurch gekennzeichnet, dass** sie Chitosan, Glucomannan und mindestens einen Wirkstoff umfassen.

16. Nanoteilchen gemäß Anspruch 15, **dadurch gekennzeichnet, dass** sie durch das Verfahren gemäß den Ansprüchen 1 bis 11 erhältlich sind.

17. Nanoteilchen gemäß einem der Ansprüche 15 und 16, **dadurch gekennzeichnet, dass** sie weiterhin ein anionisches Salz umfassen.

18. Nanoteilchen gemäß Anspruch 17, **dadurch gekennzeichnet, dass** das anionische Salz Natriumtripolyphosphat ist.

19. Nanoteilchen gemäß einem der Ansprüche 15 bis 18, **dadurch gekennzeichnet, dass** der Wirkstoff ein bioaktives Makromolekül ist.

20. Nanoteilchen gemäß einem der Ansprüche 15 bis 19, **dadurch gekennzeichnet, dass** der wirkstoff aus der Gruppe gewählt wird, umfassend Insulin, Rinderserumalbumin und immunogene Proteine.

21. Nanoteilchen gemäß einem der Ansprüche 15 bis 18, **dadurch gekennzeichnet, dass** der wirkstoff ein niedermolekulares Arzneimittel ist.

22. Nanoteilchen gemäß einem der Ansprüche 15 bis 18 und 21, **dadurch gekennzeichnet, dass** der Wirkstoff gewählt ist aus der Gruppe, umfassend Acyclovir und Indomethacin.

23. Nanoteilchen gemäß einem der Ansprüche 15 bis 22, **dadurch gekennzeichnet, dass** das Chitosan zu Glucomannan-Verhältnis zwischen 1:0,02 und 1:100 liegt.

24. Nanoteilchen gemäß einem der Ansprüche 15 bis 23, **dadurch gekennzeichnet, dass** das Chitosan zu Glucosan-Verhältnis zwischen 1:0,5 und 1:50 liegt.

25. Nanoteilchen gemäß einem der Ansprüche 15 bis 22, **dadurch gekennzeichnet, dass** sie lyophilisiert werden, nachdem sie erhalten wurden.

26. Pharmazeutische Zusammensetzung, **dadurch gekennzeichnet, dass** sie die Nanoteilchen gemäß einem der Ansprüche 15 bis 24 und mindestens ein pharmazeutisch annehmbares Exzipienz umfasst.

27. Kosmetische Zusammensetzung, **dadurch gekennzeichnet, dass** sie die Nanoteilchen gemäß einem der Ansprüche 15 bis 24 und mindestens ein kosmetisch annehmbares Exzipienz umfasst.

28. Pharmazeutische Zusammensetzung, **dadurch gekennzeichnet, dass** sie die Nanoteilchen gemäß Anspruch 25 nach Regeneration durch die Zugabe von Wasser und mindestens ein pharmazeutisch annehmbares Exzipienz umfasst.

29. Kosmetische Zusammensetzung, **dadurch gekennzeichnet, dass** sie die Nanoteilchen gemäß Anspruch 25 nach Regeneration durch die Zugabe von Wasser und mindestens ein kosmetisch annehmbares Exzipienz umfasst.

## Revendications

1. Un procédé pour l'élaboration de nanoparticules avec un diamètre moyen inférieur ou égal à 1 µm, et incorporant au moins un principe actif, **caractérisé en ce que** il comprend les étapes suivantes :
a) préparer une solution aqueuse de chitosan,
b) préparer une solution aqueuse de glucomannan, et
c) mélanger, sous agitation, les solutions des étapes a) et b), de sorte que des particules de chitosan et de glucomannan soient obtenues,
dans lequel au moins une des solutions des étapes a) et b) contient au moins un principe actif.

2. Un procédé pour l'élaboration de nanoparticules selon la revendication 1, **caractérisé en ce que** la solution de glucomannan contient un sel anionique.

3. Un procédé pour l'élaboration de nanoparticules selon la revendication 2, **caractérisé en ce que** le sel anionique est le tripolyphosphate de sodium.

4. Un procédé pour l'élaboration de nanoparticules selon la revendication 3, **caractérisé en ce que** le tripolyphosphate de sodium est à une concentration entre 0,1 et 5 mg/mL.

5. Un procédé pour l'élaboration de nanoparticules selon l'une quelconque des revendications 1 à 4, **caractérisé en ce que** la concentration de la solution de chitosan est dans un domaine de valeur entre 0,5 et 5 mg/mL.

6. Un procédé pour l'élaboration de nanoparticules selon l'une quelconque des revendications 1 à 5, **caractérisé en ce que** la concentration de la solution de glucomannan est dans un domaine de valeur entre 0,5 et 50 mg/mL.

7. Un procédé pour l'élaboration de nanoparticules selon l'une quelconque des revendications 1 à 4, **caractérisé en ce que** le rapport entre le chitosan et le glucomannan est entre 1:0,1 et 1:100.

8. Un procédé pour l'élaboration de nanoparticules selon l'une quelconque des revendications 1 à 4 et 7, **caractérisé en ce que** le rapport entre le chitosan et le glucomannan est entre 1:0,5 et 1:50.

9. Un procédé pour l'élaboration de nanoparticules selon l'une quelconque des revendications 1 à 8, **caractérisé en ce que** la solution de chitosan a un pH entre 2 et 6.

10. Un procédé pour l'élaboration de nanoparticules selon l'une quelconque des revendications 1 à 9, **caractérisé en ce que** le principe actif est une macromolécule bioactive.

11. Un procédé pour l'élaboration de nanoparticules selon l'une quelconque des revendications 1 à 10, **caractérisé en ce que** le principe actif est choisi parmi le groupe comprenant l'insuline, la sérum-albumine bovine et les protéines immunogènes.

12. Un procédé pour l'élaboration de nanoparticules selon l'une quelconque des revendications 1 à 9, **caractérisé en ce que** le principe actif est une drogue de faible poids moléculaire.

13. Un procédé pour l'élaboration de nanoparticules selon l'une quelconque des revendications 1 à 9 et 12, **caractérisé en ce que** le principe actif est choisi parmi le groupe comprenant l'acyclovir et l'indométhacine.

14. Un procédé pour l'élaboration de nanoparticules selon l'une quelconque des revendications 1 à 13, **caractérisé en ce qu'**il comprend une étape supplémentaire après l'étape c), dans laquelle les nanoparticules sont lyophilisées.

15. Nanoparticules avec un diamètre inférieur ou égal à 1 µm, pour l'administration d'au moins un principe actif, **caractérisées en ce qu'**elles comprennent du chitosan, du glucomannan et au moins un principe actif.

16. Nanoparticules selon la revendication 15, **caractérisées en ce qu'**elles sont obtenues par le procédé selon les revendications 1 à 11.

17. Nanoparticules selon l'une quelconque des revendications 15 et 16, **caractérisées en ce qu'**elles comprennent de plus un sel anionique.

18. Nanoparticules selon la revendication 17, **caractérisées en ce que** le sel anionique est le tripolyphosphate de sodium.

19. Nanoparticules selon l'une quelconque des revendications 15 à 18, **caractérisées en ce que** le principe actif est une macromolécule bioactive.

20. Nanoparticules selon l'une quelconque des revendications 15 à 19, **caractérisées en ce que** le principe actif est choisi parmi le groupe comprenant l'insuline, la sérum-albumine bovine et les protéines immunogènes.

21. Nanoparticules selon l'une quelconque des revendications 15 à 18, **caractérisées en ce que** le principe actif est une drogue de faible poids moléculaire.

22. Nanoparticules selon l'une quelconque des revendications 15 à 18 et 21, **caractérisées en ce que** le principe actif est choisi parmi le groupe comprenant l'acyclovir et l'indométhacine.

23. Nanoparticules selon l'une quelconque des revendications 15 à 22, **caractérisées en ce que** le rapport chitosan:glucomannan est entre 1:0,02 et 1:100.

24. Nanoparticules selon l'une quelconque des revendications 15 à 23, **caractérisées en ce que** le rapport chitosan:glucomannan est entre 1:0,5 et 1:50.

25. Nanoparticules selon l'une quelconque des revendications 15 à 22, **caractérisées en ce qu'**elles sont lyophilisées après leur obtention.

26. Une composition pharmaceutique, **caractérisée en ce qu'**elle comprend les nanoparticules selon l'une quelconque des revendications 15 à 24 et au moins un excipient pharmaceutiquement acceptable.

27. Une composition cosmétique, **caractérisée en ce qu'**elle comprend les nanoparticules selon l'une quelconque des revendications 15 à 24 et au moins un excipient cosmétiquement acceptable.

28. Une composition pharmaceutique, **caractérisée en ce qu'**elle comprend les nanoparticules de la revendication 25, après qu'elles aient été régénérées par addition d'eau, et au moins un excipient pharmaceutiquement acceptable.

29. Une composition cosmétique, **caractérisée en ce qu'**elle comprend les nanoparticules de la revendication 25, après qu'elles aient été régénérées par addition d'eau, et au moins un excipient cosmétiquement acceptable.
